(19) Europäisches Patentamt
European Patent Office
Office européen des brevets

(11) Veröffentlichungsnummer: **0 129 771**
**B1**

(12) **EUROPÄISCHE PATENTSCHRIFT**

(45) Veröffentlichungstag der Patentschrift: **05.10.88**

(51) Int. Cl.⁴: **C 07 D 249/08**

(21) Anmeldenummer: **84106709.3**

(22) Anmeldetag: **13.06.84**

(54) **Verfahren zur Herstellung von E-Isomeren von 1-Cyclo-hexyl-2-(1,2,4-triazol-1-yl)-1-penten-3-on-Derivaten.**

(30) Priorität: **24.06.83 DE 3322818**

(43) Veröffentlichungstag der Anmeldung:
**02.01.85 Patentblatt 85/1**

(45) Bekanntmachung des Hinweises auf die Patenterteilung:
**05.10.88 Patentblatt 88/40**

(84) Benannte Vertragsstaaten:
**BE CH DE FR GB IT LI NL**

(56) Entgegenhaltungen:
**EP - A - 0 015 387**
**EP - A - 0 057 357**
**EP - A - 0 101 996**
**US - A - 4 486 218**

(73) Patentinhaber: **BAYER AG, Konzernverwaltung RP Patentabteilung, D-5090 Leverkusen 1 Bayerwerk (DE)**

(72) Erfinder: **Feyen, Peter, Dr., Mozartstrasse 1, D-4020 Mettmann (DE)**
Erfinder: **Rauleder, Gebhard, Dr., Mozartstrasse 20, D-5657 Haan 1 (DE)**
Erfinder: **Reiser, Wolf, Dr., Kiebitzweg 12a, D-5600 Wuppertal 1 (DE)**

**EP 0 129 771 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft ein neues Verfahren zur Herstellung von bekannten pflanzenwachstumsregulierend und fungizid wirksamen E-Isomeren* von 1-Cyclo-hexyl-2-(1,2,4-triazol-1-yl)-1-penten-3-on-Derivaten.

Es ist bereits bekannt geworden, dass man 1-Cyclohexyl-2-(1,2,4-triazol-1-yl)-penten-3-on-Derivate erhält, wenn man Cyclohexanaldehyd mit entsprechenden Triazolylketonen in Gegenwart eines basischen Katalysators, wie z.B. Piperidinacetat, und in Gegenwart eines inerten organischen Lösungsmittels, insbesondere eines (aromatischen) Kohlenwasserstoffes, bei der Siedetemperatur des jeweiligen Lösungsmittels umsetzt.

Die Isolierung der Endprodukte, und hier insbesondere die Isolierung der verschiedenen Isomeren erfolgt vorzugsweise über ihre Säureadditionssalze (vergleiche EP-A-0 015 387 und US-A-4 486 218).

Dieses Verfahren hat den Nachteil, dass die Herstellung von reinen Isomeren, wie insbesondere der E-Isomeren, in wirtschaftlich ungünstiger Art und Weise verläuft. So erfordert das Freisetzen des jeweiligen reinen Isomeren aus dem entsprechenden Säureadditionssalz mit Hilfe einer Base, wie zum Beispiel Natriumcarbonat, einen Lösungsmittelwechsel und die Ausbeuten an gewünschtem isomeren Produkt sind allgemein nicht befriedigend.

Ausserdem wird in der EP-A-0 101 996 ein Verfahren zur Herstellung von E-Isomeren von Derivaten des 1-Cyclohexyl-2-(1,2,4-triazol-1-yl)-pentan-3-ons beschrieben, wobei ein vorgegebenes Gemisch von E- und Z-Isomeren mit Hilfe von sekundären Aminen umgesetzt wird.

Es wurde nun gefunden, dass man die bekannten E-Isomeren von 1-Cyclohexyl-2-(1,2,4-triazol-1-yl)-1-penten-3-on-Derivaten der Formel

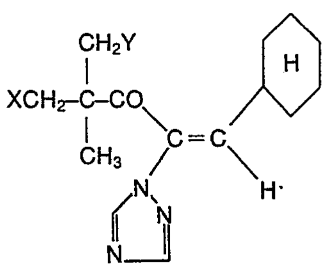

(I)

in welcher

X und Y unabhängig voneinander für Wasserstoff, Fluor, Chlor oder Brom stehen,

dadurch gekennzeichnet, dass man Triazolketone der Formel

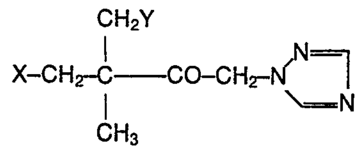

(II)

in welcher

X und Y die oben angegebene Bedeutung haben, mit Cyclohexan-aldehyd der Formel

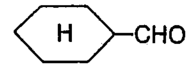

(III)

in Gegenwart eines unpolaren organischen Verdünnungsmittels sowie in Gegenwart eines Katalysator-Gemisches von organischen Säuren und sekundären Aminen in einem Molverhältnis von 1 : 0,5 bis 1 : 1 unter kontinuierlicher Entfernung des entstehenden Wassers bei Temperaturen zwischen 40 und 100 °C umsetzt, wobei das Verhältnis der Reaktionskomponenten und die Mengen an Katalysatorgemisch so gewählt werden, dass auf 1 Mol an Triazolylketon der Formel (II) 1,1 bis 1,8 Mol an Cyclohexanaldehyd der Formel (III), sowie 0,05 bis 0,5 Mol an organischer Säure und 0,05 bis 0,3 Mol an sekundärem Amin vorhanden sind, und anschliessend das Reaktionsgemisch mit wässriger Base versetzt, dann nach gegebenenfalls vorheriger Abtrennung der wässrigen Phase bei Temperaturen zwischen –20 °C und +30 °C rührt und das dabei kristallin anfallende Produkt abtrennt.

Es ist als äusserst überraschend zu bezeichnen, dass sich die E-Isomeren von 1-Cyclohexyl-2-(1,2,4-triazol-1-yl)-1-penten-3-on-Derivaten der Formel (I) nach dem erfindungsgemässen Verfahren in wesentlich höheren Ausbeuten herstellen lassen als nach dem bisher bekannten Verfahren zur Synthese dieser Stoffe.

Das erfindungsgemässe Verfahren besitzt gegenüber der vorbekannten Methode zur Herstellung der E-Isomeren von Verbindungen der Formel (I) eine Reihe von Vorteilen. So werden bei dem erfindungsgemässen Verfahren nur relativ kurze Reaktionszeiten und verhältnismässig geringe Mengen an Lösungsmittel benötigt. Ausserdem ist zwischen der eigentlichen Umsetzung und der Aufarbeitung kein Wechsel des Lösungsmittels erforderlich. Die gewünschten Produkte werden in sehr hohen Ausbeuten und in ausgezeichneter Reinheit nahezu frei von störenden Nebenprodukten erhalten. Im übrigen bereitet die Isolierung der erfindungsgemäss hergestellten Stoffe keinerlei Probleme, denn sie lassen sich durch einfaches Abfiltrieren oder Absaugen aus dem Reaktionsgemisch abtrennen. Ein weiterer entscheidender Vorteil des erfindungsgemässen Verfahrens besteht schliesslich darin, dass nicht umgesetzter Cyclohexanaldehyd und das als Katalysator dienende Gemisch aus organischer Säure und sekundärem Amin zurückgewonnen werden kann.

Die nach dem erfindungsgemässen Verfahren herstellbaren E-Isomeren von 1-Cyclohexyl-2-(1,2,4-triazol-1-yl)-1-penten-3-on-Derivaten sind durch die Formel (I) definiert.

---

* Unter «E-Isomeren» sind im vorliegenden Fall diejenigen 1-Cyclohexyl-2-(1,2,4-triazol-1-yl)-1-penten-3-on-Derivate zu verstehen, in denen der Cyclohexyl-Rest und der 1,2,4-Triazolyl-Rest auf engegengesetzten Seiten der Doppelbindung stehen.

Besonders bevorzugt sind diejenigen Verbindungen der Formel (I), in denen X für Wasserstoff steht und Y für Wasserstoff, Fluor oder Chlor steht.

Die erfindungsgemäss herstellbaren Verbindungen und deren pflanzenwuchsregulierende bzw. fungizide Eigenschaften sowie auch der Einsatz dieser Stoffe als Zwischenprodukte zur Herstellung anderer Pflanzenwachstumsregulatoren und Fungiziden sind bekannt (vgl. EP-A-0 015 387).

Verwendet man 3,3-Dimethyl-1-(1,2,4-triazol-1-yl)-butan-2-on und Cyclohexanaldehyd als Ausgangsstoffe, ein Gemisch von Piperidin und Benzoesäure als Katalysator, Isododecan als Verdünnungsmittel und Dimethylamin als sekundäres Amin bei der Kristallisation, so kann der Verlauf des erfindungsgemässen Verfahrens durch das folgende Formelschema wiedergegeben werden:

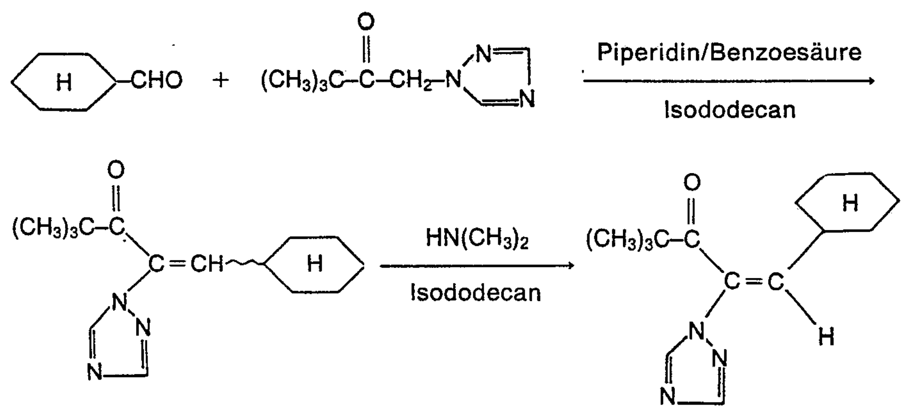


(E/Z–Isomerengemisch) (E–Isomeres)

Die bei der Durchführung des erfindungsgemässen Verfahrens als Ausgangsstoffe benötigten Triazolylketone sind durch die Formel (II) definiert. In dieser Formel stehen X und Y bevorzugt für diejenigen Reste, die bereits im Zusammenhang mit der Beschreibung der erfindungsgemäss herstellbaren Stoffe der Formel (I) bevorzugt für diese Substituenten genannt wurden.

Die Triazolylketone der Formel (II) sind bekannt (vgl. EP-A-0 015 387).

Der bei der Durchführung des erfindungsgemässen Verfahrens als Reaktionskomponente benötigte Cyclohexanaldehyd der Formel (III) ist ebenfalls bekannt.

Als Verdünnungsmittel können bei der Durchführung des erfindungsgemässen Verfahrens alle unpolaren organischen Solventien eingesetzt werden. Vorzugsweise in Frage kommen Xylol, Toluol, Cyclohexan, Ligroin, Isooctan, Dodecan und Isododecan.

Bei dem erfindungsgemässen Verfahren dient ein Gemisch aus einer organischen Säure und einem sekundären Amin als Katalysator. Vorzugsweise verwendbare organische Säuren sind hierbei Essigsäure, Propionsäure, Pivalinsäure, 2-Ethyl-hexansäure, Cyclohexancarbonsäure und Benzoesäure. Als sekundäre Amine kommen vorzugsweise Dimethylamin, Diethylamin, Di-isopropylamin, Di-isobutylamin, Pyrrolidin, Morpholin, Hexahydro-1-H-azepin, Piperidin, 2-Methyl-piperidin und 2,6-Dimethylpiperidin in Betracht.

In dem Katalysatorgemisch kann das Verhältnis von organischer Säure zu sekundärem Amin innerhalb eines bestimmten Bereiches variiert werden. Vorzugsweise verwendet man auf 1 Mol an organischer Säure 0,5 bis 0,9 Mol an sekundärem Amin.

Die Reaktionstemperaturen können bei der erfindungsgemässen Umsetzung innerhalb eines bestimmten Bereiches variiert werden. Vorzugsweise arbeitet man bei Temperaturen zwischen 50 °C und 80 °C.

Bei der Durchführung des erfindungsgemässen Verfahrens arbeitet man entweder bei Normaldruck oder auch unter vermindertem Druck, z.B. bei Drucken zwischen 5 und 100 mbar, vorzugsweise zwischen 10 und 50 mbar. Letzteres ist vor allem dann zweckmässig, wenn man bei relativ niedrigen Reaktionstemperaturen und mit verhältnismässig hoch siedenden Lösungsmitteln arbeitet.

Während der Durchführung der erfindungsgemässen Umsetzung wird das entstehende Wasser kontinuierlich aus dem Reaktionsgemisch entfernt. Im allgemeinen geschieht dieses mit Hilfe eines Wasserabscheiders. Es ist jedoch auch möglich, das sich bildende Wasser im Reaktionsgemisch durch Zugabe geeigneter Materialien, wie z.B. von Molekularsieben, zu binden.

Die Reaktionszeiten können bei der erfindungsgemässen Umsetzung innerhalb eines grösseren Bereiches variiert werden. Im allgemeinen beträgt die Reaktionsdauer zwischen 3 und 20 Stunden, vorzugsweise zwischen 4 und 14 Stunden.

Bei der Durchführung des erfindungsgemässen Verfahrens setzt man die Triazolylketone der Formel (II) mit einem Überschuss an Cyclohexanaldehyd der Formel (III) um. Vorzugsweise setzt man auf 1 Mol an Triazolylketon der Formel (II) 1,15 bis 1,6 Mol an Cyclohexanaldehyd der Formel (III) ein.

Die Menge an Katalysator-Gemisch kann ebenfalls innerhalb eines bestimmten Bereichs variiert werden. Vorzugsweise setzt man auf 1 Mol an Triazolylketon der Formel (II) zwischen 0,1 und 0,3 Mol an organischer Säure und zwischen 0,05 und 0,2 Mol an sekundärem Amin ein. Auch die Menge an unpolarem organischen Verdünnungsmittel kann innerhalb eines bestimmten Bereiches variiert werden. Das Verfahren kann sogar in Abwesenheit eines Lösungsmittels durchgeführt werden. Im allgemeinen verwendet man auf 1 Mol an Triazolylketon der Formel (II) 0 bis 1200 g, vorzugsweise 50 bis 500 g an Lösungsmittel.

Im einzelnen verfährt man bei der Durchführung der erfindungsgemässen Umsetzung in der Weise, dass man die Ausgangsstoffe, die Katalysatorkomponenten und das Lösungsmittel in den angegebenen Mengen zusammengibt und durch Rühren und/oder Kochen, gegebenenfalls unter vermindertem Druck, für eine gute Durchmischung sorgt. Dabei hat es sich als zweckmässig erwiesen, das Triazolylketon der Formel (II), die Katalysator-Säure und das unpolare Lösungsmittel vorzulegen und eine Mischung aus Cyclohexanaldehyd der Formel (III) und Katalysator-Base (sekundäres Amin) innerhalb eines Zeitraumes von 0,5 bis zu 2 Stunden hinzuzugeben.

Es kann technisch zweckmässig sein, aus der Mischung Cyclohexanaldehyd und Katalysator-Base zunächst eine Teilmenge Wasser auszukreisen und dann die entwässerte Mischung zu dosieren.

Nach beendeter Umsetzung und Abkühlung des Reaktionsgemisches auf 20 bis 40 °C erfolgt die Aufarbeitung.

Im allgemeinen geht man so vor, dass man Reaktionsgemisch mit wässriger Base, vorzugsweise verdünnter wässriger Alkalibase, wie z.B. Natronlauge oder Kalilauge, versetzt, gut durchmischt und gegebenenfalls nach vorherigem Abtrennen der wässrigen Phase unter intensivem Rühren auf Temperaturen zwischen −20 °C und +30 °C, vorzugsweise zwischen −10 °C und +10 °C abkühlt. Die gewünschten Produkte scheiden sich dabei kristallin ab und können durch Filtration oder Absaugen abgetrennt und durch Waschen mit wenig kaltem organischem Lösungsmittel und anschliessendem Trocknen in reiner Form isoliert werden. Gegebenenfalls können die Produkte durch Umkristallisation von evtl. noch vorhandenen Verunreinigungen befreit werden. Aus dem nach der Isolierung des kristallinen Produktes verbleibenden Filtrat kann nach dem aufeinanderfolgenden Waschen mit wässriger Mineralsäure, wie z.B. Schwefelsäure, verdünnter wässriger Base, wie z.B. Natronlauge oder Kalilauge und Wasser sowie nach weiterem Einengen der organischen Phase gegebenenfalls nach Zugabe eines sekundären Amins, wie z.B. Dimethylamin oder Piperidin, durch Rühren bei niedriger Temperatur zusätzliches Produkt in kristalliner Form gewonnen werden.

Bei dem erfindungsgemässen Verfahren abdestillierter Cyclohexanaldehyd und Lösungsmittel können erneut zur Reaktion eingesetzt werden. Auch die Katalysator-Komponenten lassen sich in einfacher Weise isolieren und wieder verwenden. Dabei geht man im allgemeinen so vor, das man die vereinigten wässrigen Phasen zunächst durch Zugabe von wässriger Alkalilauge, z.B. Natronlauge oder Kalilauge, auf einen pH-Wert von 12 einstellt und sekundäres Amin im Gemisch mit Wasser destillativ abtrennt. Neben der destillativen Abtrennung des Amins kann das Amin auch extraktiv entfernt werden, wobei als Extraktionsmittel zweckmässigerweise eine Mischung aus Cyclohexanaldehyd und dem zur Reaktion verwendeten Lösungsmittel verwendet wird. Nach Ergänzung von Aminverlusten können die Extrakte direkt zur erneuten Umsetzung eingesetzt werden. Der verbleibende Destillationsdampf wird dann durch Zugabe von wässriger Mineralsäure, z.B. Schwefelsäure, auf einen pH-Wert zwischen 1 und 4 gebracht, so dass die Katalysator-Säure freigesetzt wird und entweder durch Destillation oder Extraktion oder, – sofern es sich um einen Feststoff handelt –, auch durch Absaugen isoliert werden kann.

Das erfindungsgemässe Verfahren kann entweder diskontinuierlich oder auch kontinuierlich durchgeführt werden.

Das erfindungsgemässe Verfahren wird durch die folgenden Beispiele veranschaulicht.

Vergleichsbeispiel

Herstellung des E-Isomeren des 1-Cyclohexyl-4,4-dimethyl-2-(1,2,4-triazol-1-yl)-1-penten-3-ons der Formel

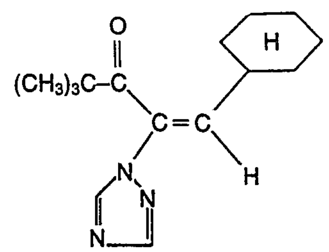

nach dem Verfahren gemäss EP-A-0 015 387.

Ein Gemisch aus 83,5 g (0,5 Mol) 3,3-Dimethyl-1-(1,2,4-triazol-1-yl)-butan-2-on, 60 g (0,54 Mol) Cyclohexanaldehyd, 4,2 g (0,05 Mol) Piperidin und 6 g (0,1 Mol) Eisessig in 300 ml Toluol wurde am Wasserabscheider so lange unter Rückfluss erhitzt, bis sich kein Wasser mehr abschied. Nach dem Abkühlen auf Raumtemperatur wurde das Reaktionsgemisch mit gesättigter wässriger Natriumchlorid-Lösung gewaschen. Die organische Phase wurde nach dem Trocknen über Natriumsulfat filtriert und durch Abziehen des Lösungsmittels unter vermindertem Druck eingeengt. Der verbleibende Rückstand wurde in 500 ml Aceton aufgenommen und unter Rühren mit einer filtrierten Lösung von 90 g (0,25 Mol) Naphthalin-1,5-disulfonsäure in 500 ml Aceton versetzt.

Der zunächst ausfallende Niederschlag wurde abgesaugt, das Filtrat weiter eingeengt und der erhaltene farblose kristalline Rückstand in 500 ml Methylenchlorid aufgenommen. Danach wurde halbkonzentrierte wässrige Natriumcarbonat-

Lösung bis zur alkalischen Reaktion zugegeben. Die organische Phase wurde abgetrennt, getrocknet, filtriert und eingeengt. Der ölige Rückstand wurde in Petrolether aufgenommen, und der Kristallisation überlassen. Man erhielt 64 g (49% der Theorie) an E-Isomeren des 1-Cyclohexyl-4,4-dimethyl-2-(1,2,4-triazol-1-yl)-1-penten-3-ons in Form einer Festsubstanz vom Schmelzpunkt 98 °C.

Erfindungsgemässe Herstellungsbeispiele

Beispiel 1

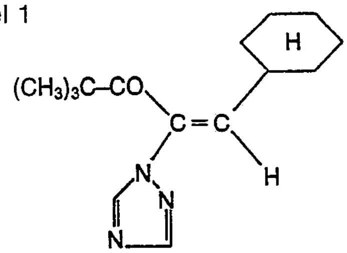

E-Isomeres

Ein Gemisch aus 1 Mol 3,3-Dimethyl-1-(1,2,4-triazol-1-yl)-butan-3-on, 0,45 Mol Benzoesäure, 1,3 Mol Cyclohexanaldehyd, 0,3 Mol Piperidin und 300 g Isododecan wurde 8 Stunden bei 62–65 °C unter einem Druck von etwa 20 mbar am Wasserabscheider gekocht. Nachdem sich 22,5 ml Wasser abgeschieden hatten, wurde aufgearbeitet, indem man die Apparatur mit Stickstoff belüftete, das Reaktionsgemisch mit 100 g 20%iger wässriger Natronlauge wusch, dann auf −10 °C abkühlte und 4 Stunden bei dieser Temperatur intensiv rührte. Die dabei entstandenen farblosen Kristalle wurden abgesaugt, mit kaltem Isododecan gewaschen und unter vermindertem Druck bei 50 °C getrocknet. Man erhielt auf diese Weise ein Produkt, das zu 96% aus dem E-Isomeren des 1-Cyclohexyl-4,4-dimethyl-2-(1,2,4-triazol-1-yl)-1-penten-3-ons bestand. Die Ausbeute betrug 75% der Theorie.

Die Mutterlauge wurde anschliessend zunächst mit 100 g Wasser und konzentrierter Salzsäure gewaschen und dann mit 100 g Wasser und einigen Tropfen 20%iger Natronlauge neutral gewaschen. Danach wurde Cyclohexanaldehyd und etwas Lösungsmittel abdestilliert und bei Temperaturen zwischen −5 °C und 0 °C mit Dimethylamin versetzt. Die kristalline Festsubstanz, die sich nach mehrstündigem Rühren bei −5 °C bis 0 °C abgeschieden hatte, wurde abgesaugt, mit wenig Isododecan gewaschen und getrocknet. Man erhielt auf diese Weise weitere 36 g eines Produktes, das zu 96% aus dem E-Isomeren des 1-Cyclohexyl-4,4-dimethyl-2-(1,2,4-triazol-1-yl)-1-penten-3-ons bestand. Die Gesamtausbeute betrug 87% der Theorie.

Beispiel 2

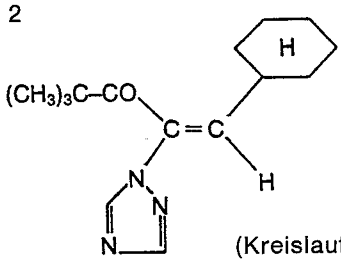

E-Isomeres

(Kreislauf-Verfahren)

Ein im Kreislauf geführtes Gemisch aus 25,2 g (0,3 Mol) Piperidin, 34,0 g (0,3 Mol) Cyclohexanaldehyd und 300 g Isododecan wurde mit 1 Mol 3,3-Dimethyl-1-(1,2,4-triazol-1-yl)-butan-3-on, 1 Mol Cyclohexanaldehyd und 54,9 g (0,45 Mol) recyclisierter Benzoesäure versetzt und 8 Stunden unter einem Druck von 15–20 mbar am Wasserabscheider gekocht. Nach dem anschliessenden Belüften der Apparatur mit Stickstoff wurde das Reaktionsgemisch mit einer 10%igen wässrigen Natronlauge versetzt, die 0,5 Mol an Natriumhydroxid enthielt. Dabei stammte ein Teil des zur Herstellung der Natronlauge benötigten Wassers aus dem Teil des vorausgegangenen Ansatzes, aus dem das Piperidin zurückgewonnen wurde. Die wässrige Phase wurde abgetrennt und durch kurzzeitiges Erhitzen unter vermindertem Druck andestilliert, wobei in der Vorlage ein Gemisch aus Piperidin und Wasser aufgefangen wurde, das im nächsten Cyclus des Kreislauf-Verfahrens bei der Wäsche mit Natronlauge wieder eingesetzt wurde. Aus dem verbleibenden Sumpfprodukt wurde durch Ansäuern mit Salzsäure bis zu einem pH-Wert von 2 die Benzoesäure ausgefällt, abgesaugt und im nächsten Ansatz wieder verwendet. Die organische Phase wurde abgekühlt und 4 Stunden bei −10 °C bis 0 °C gerührt. Der anfallende Niederschlag wurde abgesaugt, mit kaltem Isododecan gewaschen und getrocknet. Man erhielt auf diese Weise das E-Isomere des 1-Cyclohexyl-4,4-dimethyl-2-(1,2,4-triazol-1-yl)-1-penten-3-ons in einer Ausbeute von 85% der Theorie.

Die nach dem Absaugen des E-Isomeren des 1-Cyclohexyl-4,4-dimethyl-2-(1,2,4-triazol-1-yl)-1-penten-3-ons verbleibende Mutterlauge wurde nach Entnahme einer Purge-Stromes mit dem bei der Reaktion verbrauchten Anteil an Cyclohexanaldehyd und 3,3-Dimethyl-1-(1,2,4-triazol-1-yl)-butan-3-on versetzt und nach Zugabe von recyclisierter Benzoesäure erneut in den Kreislauf-Prozess eingeführt.

Beispiel 3

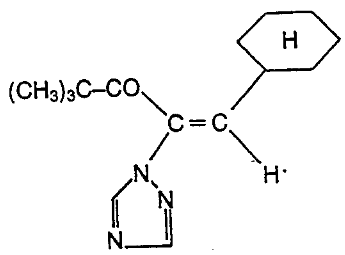

E-Isomeres

Das Verfahren wurde in einer 6 Liter fassenden Rührapparatur mit externem Umpumpkreislauf durchgeführt, wobei sich in dem externen Umpumpkreislauf eine mit 1,6 kg Zeolith gefüllte Glassäule befand. Das Umpumpen des Materials wurde mit Hilfe einer Zahnradpunpe erreicht; das Material wurde in einer Dosierung von etwa 25–30 Liter/Stunde von unten in die Glassäule eingeführt.

Die folgenden Komponenten wurden nacheinander in die Apparatur gegeben:

1377 g (8,0 Mol) 3,3-Dimethyl-1-(1,2,4-triazol-1-yl)-butan-3-on (Reinheitsgrad 97%),
1070 g (9,36 Mol) Cyclohexancarbaldehyd (98%ig),
180 g (1,47 Mol) Benzoesäure,
84 g (0,98 Mol) Piperidin,
1400 g Toluol

Das Gesamtvolumen betrug etwa 4,1 Liter bei 70 °C.

Die Mischung wurde unter Rühren mit Dampf auf eine Temperatur von 65 °C aufgeheizt. Nach dem Lösen der Feststoffe wurde das Umpumpwerk eingeschaltet, und das Reaktionsgemisch wurde 9 Stunden auf einer Temperatur von 69 bis 71 °C gehalten. Danach wurde das Reaktionsgemisch in einen separaten Kolben zum Waschen gegeben. Das Zeolith-Material wurde zunächst 30 Minuten lang bei einer Temperatur von 70 °C mit 1200 ml Toluol extrahiert. Diese Toluol-Lösung wurde mit dem Reaktionsgemisch vereinigt. Anschliessend wurde das Zeolith-Material noch zweimal mit je 1200 ml Methanol in gleicher Weise gespült. Die vereinigten Methanol-Phasen wurden eingeengt, und der verbleibende Rückstand wurde in den Waschkolben überführt, wo nacheinander die folgenden Operationen vorgenommen wurden:

1. Alkalische Wäsche mit 1200 g 5%iger wässriger Natronlauge.
2. Saure Wäsche mit 10%iger wässriger Schwefelsäure bis zu einem pH-Wert von 3 bis 4.
3. Neutrale Wäsche mit 1000 g Wasser.

Die organische Phase wurde am Dünnschicht-Verdampfer ganz eingeengt, und der verbleibende Rückstand wurde bei Raumtemperatur innerhalb von 1 Minute unter kräftigem Rühren mit einer Mischung aus

2640 g Wasser
264 g einer 40%igen wässrigen Dimethylamin-Lösung und
264 g eines Emulgators der Formel

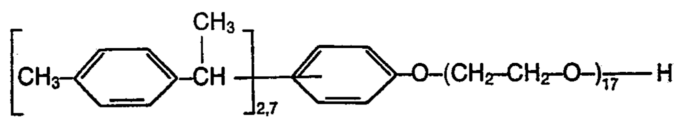

versetzt. Die entstehende Mischung wurde 4 Stunden auf einer Temperatur von 20 bis 22 °C gehalten und dann auf 5 °C abgekühlt. Der dabei ausfallende farblose Niederschlag wurde abgesaugt, zweimal mit je 1080 ml kaltem Wasser gewaschen und getrocknet. Man erhielt auf diese Weise 1856 g (88% der Theorie) an dem E-Isomeren des 1-Cyclohexyl-4,4-dimethyl-2-(1,2,4-triazol-1-yl)-1-penten-3-ons mit einem Reinheitsgrad von 99%.

**Patentansprüche**

1. Verfahren zur Herstellung von E-Isomeren von 1-Cyclohexyl-2-(1,2,4-triazol-1-yl)-1-penten-3-on-Derivaten der Formel

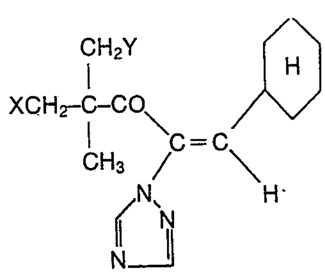 (I)

in welcher
X und Y unabhängig voneinander für Wasserstoff, Fluor, Chlor oder Brom stehen,
dadurch gekennzeichnet, dass man Triazolylketone der Formel

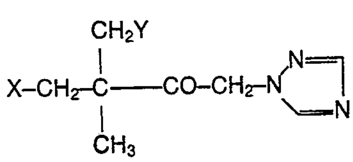 (II)

in welcher
X und Y die oben angegebene Bedeutung haben, mit Cyclohexan-aldehyd der Formel

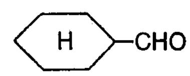 (III)

in Gegenwart eines unpolaren organischen Verdünnungsmittels sowie in Gegenwart eines Katalysator-Gemisches von organischen Säuren und sekundären Aminen in einem Molverhältnis von 1 : 0,5 bis 1 : 1 unter kontinuierlicher Entfernung des entstehenden Wassers bei Temperaturen zwischen 40 und 100 °C umsetzt, wobei das Verhältnis der Reaktionskomponenten und die Mengen an Katalysatorgemisch so gewählt werden, dass auf 1 Mol an Triazolylketon der Formel (II) 1,1 bis 1,8 Mol an Cyclohexanaldehyd der Formel (III) sowie 0,05 bis 0,5 Mol an organischer Säure und 0,05 bis 0,3 Mol an sekundärem Amin vorhanden sind, und anschliessend das Reaktionsgemisch mit wässriger Base versetzt, dann nach gegebenenfalls vorheriger Abtrennung der wässrigen Phase bei Temperaturen zwischen −20 °C und +30 °C rührt und das dabei kristallin anfallende Produkt abtrennt.

2. Verfahren gemäss Anspruch 1, dadurch gekennzeichnet, dass man als Ausgangsstoffe Triazolylketone der Formel (II) einsetzt, in denen X für Wasserstoff steht und Y für Wasserstoff, Fluor oder Chlor steht.

3. Verfahren gemäss Anspruch 1, dadurch gekennzeichnet, dass man als Triazolylketon der Formel (II) 3,3-Dimethyl-1-(1,2,4-triazol-1-yl)-butan-3-on der Formel

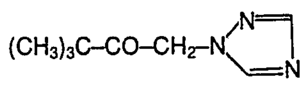

einsetzt.

4. Verfahren gemäss Anspruch 1, dadurch gekennzeichnet, dass man als Verdünnungsmittel, Xylol, Toluol, Cyclohexan, Ligroin, Isooctan, Dodecan oder Isododecan einsetzt.

5. Verfahren gemäss Anspruch 1, dadurch gekennzeichnet, dass man als Säurekomponenten des Katalysator-Gemisches Essigsäure, Propionsäure, Pivalinsäure, 2-Ethyl-hexansäure, Cyclohexancarbonsäure oder Benzoesäure einsetzt.

6. Verfahren gemäss Anspruch 1, dadurch gekennzeichnet, dass man als Amin-Komponenten des Katalysator-Gemisches Dimethylamin, Diethylamin, Di-isopropylamin, Di-iso-butyl-amin, Pyrrolidin, Morpholin, Hexahydro-1-H-azepin, Piperidin, 2-Methylpiperidin oder 2,6-Dimethyl-piperidin einsetzt.

7. Verfahren gemäss Anspruch 1, dadurch gekennzeichnet, dass man die Umsetzung bei Temperaturen zwischen 50 °C und 80 °C durchführt.

**Claims**

1. Process for preparing E-isomers of 1-cyclohexyl-2-(1,2,4-triazol-1-yl)-1-penten-3-one derivatives of the formula

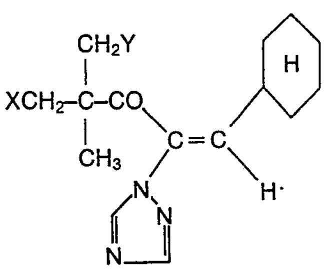

(I)

in which

X and Y independently of each other represent hydrogen, fluorine, chlorine or bromine, characterised in that triazolyl-ketones of the formula

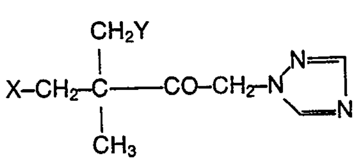

(II)

in which

X and Y have the abovementioned meaning, are reacted with cyclohexane-aldehyde of the formula

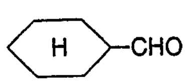

(III)

in the presence of a non-polar organic diluent and in the presence of a catalyst mixture of organic acids and secondary amines in a molar ratio of 1 : 0.5 to 1 : 1 at temperatures between 40 and 100 °C while continuously removing the water formed, the ratio of the reactants and the amounts of catalyst mixture being chosen such that 1.1 to 1.8 moles of cyclohexane-aldehyde of the formula (III) and 0.05 to 0.5 mole of organic acid and 0.05 to 0.3 mole of secondary amine are present per mole of triazolyl-ketone of the formula (II), and then afterwards an aqueous base is added to the reaction mixture, which, if appropriate after the aqueous phase has been separated off, is then stirred at temperatures between −20 °C and +30 °C, and the product precipitating in the form of crystals is separated off.

2. Process according to Claim 1, characterised in that the starting materials used are triazolyl-ketones of the formula (II) in which X represents hydrogen and Y represents hydrogen, fluorine or chlorine.

3. Process according to Claim 1, characterised in that 3,3-dimethyl-1-(1,2,4-triazol-1-yl)-butan-3-one of the formula

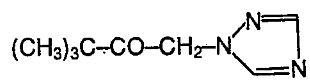

is used as the triazolyl-ketone of the formula (II).

4. Process according to Claim 1, characterised in that the diluent used is xylene, toluene, cyclohexane, ligroin, isooctane, dodecane or isododecane.

5. Process according to Claim 1, characterised in that the catalyst mixture's acid component used is acetic acid, propionic acid, pivalic acid, 2-ethylhexanoic acid, cyclohexanecarboxylic acid or benzoic acid.

6. Process according to Claim 1, characterised in that the catalyst mixture's amine component used is dimethylamine, diethylamine, diisopropylamine, diisobutylamine, pyrrolidine, morpholine, hexahydro-1-H-azepine, piperidine, 2-methylpiperidine or 2,6-dimethylpiperidine.

7. Process according to Claim 1, characterised in that the reaction is carried out at temperatures between 50 °C and 80 °C.

**Revendications**

1. Procédé pour préparer l'isomère E de dérivés de la 1-cyclohexyl-2-(1,2,4-triazol-1-yl)-1-pentène-3-one, de formule:

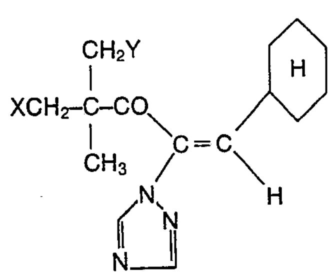

(I)

dans laquelle

X et Y représentent, indépendamment l'un de l'autre, un atome d'hydrogène, de fluor, de chlore ou de brome,

procédé caractérisé en ce qu'on fait réagir des triazolylcétones de formule:

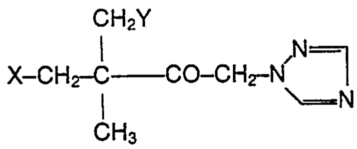

(II)

dans laquelle
X et Y ont les sens indiqué ci-dessus, avec le cyclohexane-aldéhyde de formule:

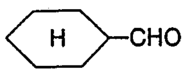

(III)

en opérant en présence d'un diluant organique non polaire ainsi qu'en présence d'un mélange, formant catalyseur, constitué d'acides organiques et d'amines secondaires en un rapport molaire de 1 : 0,5 à 1 : 1, avec élimination en continu de l'eau ainsi obtenue, à des températures comprises entre 40 et 100 °C, le rapport entre les constituants du mélange réactionnel et les quantités du mélange formant le catalyseur étant choisi de manière qu'il y ait, pour 1 mol de la triazolylcétone de formule (II) 1,1 à 1,8 mol de cyclohexane-aldéhyde de formule (III) ainsi que 0,05 à 0,5 mol d'acide organique et 0,05 à 0,3 mol d'amine secondaire, et on ajoute ensuite au mélange réactionnel une base aqueuse, et, après séparation éventuellement de la phase aqueuse, on agite ensuite à des températures comprises entre −20 °C et +30 °C et l'on sépare le produit obtenu sous forme cristalline.

2. Procédé selon la revendication 1, caractérisé en ce qu'on utilise comme substance de départ des triazolylcétones de formule (II), dans lesquelles X représente un atome d'hydrogène et Y représente un atome d'hydrogène, de fluor ou de chlore.

3. Procédé selon la revendication 1, caractérisé en ce qu'on utilise comme triazolylcétone de formule (II) la 3,3-di-méthyl-1-(1,2,4-triazol-1-yl-)-butane-3-one de formule

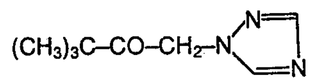

4. Procédé selon la revendication 1, caractérisé en ce qu'on utilise comme diluant le xylène, le toluène, le cyclohexane, la ligroïne, l'isooctane, le dodécane ou l'isododécane.

5. Procédé selon la revendication 1, caractérisé en ce qu'on utilise comme composants acides du mélange formant le catalyseur l'acide acétique, l'acide propionique, l'acide pivalique, l'acide 2-éthyl-hexanoïque, l'acide cyclohexanecarboxylique ou l'acide benzoïque.

6. Procédé selon la revendication 1, caractérisé en ce qu'on utilise comme composants amines du mélange formant le catalyseur la diméthylamine, la diéthylamine, la diisopropylamine, la diisobutylamine, la pyrrolidine, la morpholine, l'hexahydro-1-H-azépine, la pipéridine, la 2-méthylpipéridine ou la 2,6-diméthylpipéridine.

7. Procédé selon la revendication 1, caractérisé en ce qu'on conduit la réaction à des températures comprises entre 50 °C et 80 °C.